# FASCICULE DE BREVET EUROPEEN

(11) **EP 1 299 042 B1**
(45) Date de publication et mention de la délivrance du brevet: **22.03.2006**
(21) Numéro de dépôt: 01955412.0
(22) Date de dépôt: 12.07.2001
(51) Int. Cl.: A61B 17/70

(54) **IMPLANT INTERVERTEBRAL AMORTISSANT**
STOSSDÄMPFENDES ZWISCHENWIRBELIMPLANTAT
SHOCK-ABSORBING INTERVERTEBRAL IMPLANT

(30) Priorité: 12.07.2000 FR 0009093
(43) Date de publication de la demande: 09.04.2003
(73) Titulaire: Abbott Spine, La Cité Mondiale, 33000 Bordeaux (FR)
(72) Inventeur: LE COUEDIC, Régis, F-33000 BORDEAUX (FR); SENEGAS, Jacques, F-33700 Merignac (FR)
(74) Mandataire: Dronne, Guy
(86) Numéro de dépôt international: PCT/FR2001/002261
(87) Numéro de publication internationale: WO 2002/003882

(56) Documents cités:
- WO-A-94/26195
- WO-A-99/21501
- FR-A- 2 681 525
- FR-A- 2 717 675
- FR-A- 2 774 581
- US-A- 5 496 318

## Description

La présente invention concerne un implant intervertébral destiné à s'appliquer notamment entre deux apophyses épineuses de deux vertèbres.

Les indications pour lesquelles ce type d'implant est inséré et fixé entre les apophyses épineuses ont généralement pour origine la dégénérescence du disque intervertébral. En effet, lorsque la partie postérieure du disque intervertébrale est dégradée, notamment, l'extension du rachis provoque le rapprochement anormal des deux vertèbres séparées par le disque. Ce rapprochement induit généralement le pincement des racines nerveuses et l'apparition de la douleur chez le sujet qui est affecté par ce trouble.

Des implants intervertébraux comprenant une cale insérable entre les apophyses épineuses et comportant des moyens de fixation sont bien connus. Les cales, généralement constituées en alliage de titane, présentent une gorge à chacune de leurs extrémités, de façon que les apophyses épineuses soient calées dans les gorges. Par ailleurs, le maintien de la cale est assuré par un lien reliant les deux bords opposés de chacune des gorges et enserrant partiellement la paroi des apophyses épineuses.

De tels implants permettent de limiter le rapprochement des vertèbres puisque, lorsque le rachis est en extension, les apophyses épineuses tendent à venir en butée contre le fond des gorges opposées dans lesquelles elles sont introduites. Cependant, le matériau dans lequel est réalisée la cale est dur au regard du matériau discal qui limite le rapprochement des vertèbres lorsqu'il est intact si bien que les ondes de chocs qui peuvent être transmises au rachis, lors de la marche par exemple, ne sont pas amorties entre deux vertèbres reliées par la cale. En outre, la cale n'ayant pas des propriétés mécaniques identiques à la portion restante de disque intervertébral, les propriétés mécaniques globales du rachis présentent des discontinuités importantes au regard d'un rachis intact, ce qui accroît la dégénérescence du disque intervertébral.

Le document FR 2 717 675 décrit un implant de type connu destiné à amortir les mouvements relatifs de deux vertèbres adjacentes lors des mouvements de flexion et d'extension du rachis. Cet implant présente les caractéristiques définies dans le préambule de la revendication 1.

Un objet de la présente invention est de fournir un implant intervertébral dont les deux gorges opposées contre lesquelles les apophyses épineuses viennent en butée présentent une mobilité relative et dont les mouvements relatifs sont amortis.

Pour atteindre ce but, conformément à l'invention, l'implant intervertébral comportant une cale destinée à s'appliquer entre deux apophyses épineuses de deux vertèbres comprend :
- deux éléments constitués dans un premier matériau, présentant une première extrémité et une deuxième extrémité, ladite première extrémité étant solidarisable à une apophyse épineuse, et,
- une pièce de liaison constituée dans un deuxième matériau de plus grande déformabilité élastique que ledit premier matériau, reliant lesdites deuxièmes extrémités desdits deux éléments de sorte que les contraintes qui s'exercent sur lesdits deux éléments sont amorties, par quoi ledit implant intervertébral est apte à limiter et à freiner le mouvement relatif desdites vertèbres.

Ainsi, la pièce de liaison située entre les deux éléments, chacun solidaire d'une apophyse épineuse, tend à se comprimer lorsque les apophyses épineuses se rapprochent et à absorber les contraintes exercées sur lesdits deux éléments. De la sorte, le rapprochement des vertèbres se fait avec une certaine élasticité qui est proche de l'élasticité naturelle que confère un disque intervertébral intact. En outre, la mobilité relative élastique des vertèbres est compatible avec la déformation élastique des ligaments postérieurs qui assurent le maintien des vertèbres ensemble. On obtient alors un système dont la mobilité relative, sous contraintes, des éléments qui le constituent est sensiblement identique à la mobilité relative des éléments du système originel intact, ce qui préserve ces éléments de la progression de la dégénérescence.

Lesdites deuxièmes extrémités de la cale présentent une paroi de solidarisation sur laquelle ladite pièce de liaison est apte à adhérer. Ainsi, l'implant se caractérise en ce qu'aucun organe de fixation additionnel n'est nécessaire et en ce que les propriétés adhésives du deuxième matériau coopèrent avec la paroi de solidarisation.

Ladite paroi de solidarisation présente des évidements aptes à coopérer avec des aspérités de ladite pièce de liaison de façon à augmenter l'adhérence de ladite pièce de liaison sur ladite paroi. On comprend en effet que le fait de ménager des évidements dans une paroi accroît la surface de cette paroi, ce qui augmente la surface de contact entre les deux matériaux si l'un des matériaux peut être moulé sur la paroi de l'autre. L'augmentation de la surface de contact induit l'augmentation des forces de liaison entre ladite pièce de liaison et lesdits deux éléments. En outre, on pratique des évidements de façon à augmenter la surface de contact et à augmenter corrélativement les forces de frottement statique du matériau de la pièce de liaison sur lesdits deux éléments, ces forces s'additionnant aux forces de liaison.

Avantageusement, ledit deuxième matériau de ladite pièce de liaison est constitué d'un corps obtenu par polymérisation. De la sorte, la pièce de liaison peut facilement être moulée à chaud sur lesdits éléments si le matériau est polymérisé au préalable, ou elle peut être constituée in situ si la vitesse de polymérisation des monomères constituant ledit deuxième matériau est suffisamment faible pour disposer du temps nécessaire à la réalisation de l'assemblage.

Selon un mode préféré de mise en oeuvre de l'invention, ledit premier matériau desdits éléments est un alliage de titane. Ainsi, il est aisé de ménager des évidements dans ladite paroi de solidarisation sur laquelle ladite pièce de liaison est apte à adhérer.

Selon un premier mode particulier de réalisation, chaque première extrémité desdits deux éléments forme une gorge, définissant deux ailes, apte à recevoir une apophyse épineuse et en ce que ledit implant comprend en outre un lien de longueur réglable reliant lesdites deux ailes, ledit lien entourant une portion de ladite apophyse épineuse de façon à solidariser ladite première extrémité à ladite apophyse épineuse.

Ainsi, cette caractéristique de l'implant intervertébral réside dans le mode de fixation desdits éléments sur les apophyses épineuses. Ledit lien est pré-installé sur lesdits éléments de ladite cale et, lorsque cette dernière est insérée entre deux apophyses, le serrage du lien permet la solidarisation desdits éléments aux apophyses.

Selon un deuxième mode particulier de mise en oeuvre de l'invention chaque première extrémité desdits deux éléments forme une gorge apte à recevoir une apophyse épineuse, ladite gorge définissant deux ailes, et en ce que ledit implant comprend en outre une tige apte à traverser latéralement lesdites ailes et ladite apophyse de façon à solidariser ladite première extrémité à ladite apophyse épineuse.

Dans cette configuration, les apophyses épineuses sont percées transversalement et les éléments y sont reliés au moyen d'une tige ou d'un rivet qui traverse à la fois les deux ailes de l'élément et l'apophyse située entre les deux. La tige ou le rivet est fixé sur l'une des ailes ou sur les deux de manière éviter son retrait accidentel.

Selon un troisième mode particulier de mise en oeuvre de l'invention, chaque première extrémité forme une gorge apte à recevoir une apophyse épineuse, ladite gorge définissant deux ailes, et ledit implant comprend en outre une pièce hémicirculaire formant agrafe reliant lesdites ailes, ladite agrafe entourant une portion de ladite apophyse épineuse de façon à solidariser ladite première extrémité à ladite apophyse épineuse.

De la sorte, les agrafes sont fixables aisément sur les ailes desdits éléments, après avoir inséré la cale. Comme on fexpfiquera plus en détails, selon ce mode particulier de mise en oeuvre, les ailes étant dégagées, l'insertion de la cale est effectuée sans intervention majeure sur les ligaments postérieurs.

D'autres particularités et avantages de l'invention ressortiront à la lecture de la description faite ci-après de modes de réalisation particuliers de l'invention, donnés à titre indicatif mais non limitatif, en référence aux dessins annexés sur lesquels:
- la Figure 1 est une vue en coupe axiale d'une cale intervertébrale conforme à l'invention,
- la Figure 2, est une vue schématique en élévation montrant l'implant intervertébral muni de ses liens réglables de fixation,
- la Figure 3 est une vue schématique en élévation montrant l'implant intervertébral muni de tiges de fixation, et,
- la Figure 4 est une vue schématique en élévation montrant l'implant intervertébral muni d'agrafes de fixation

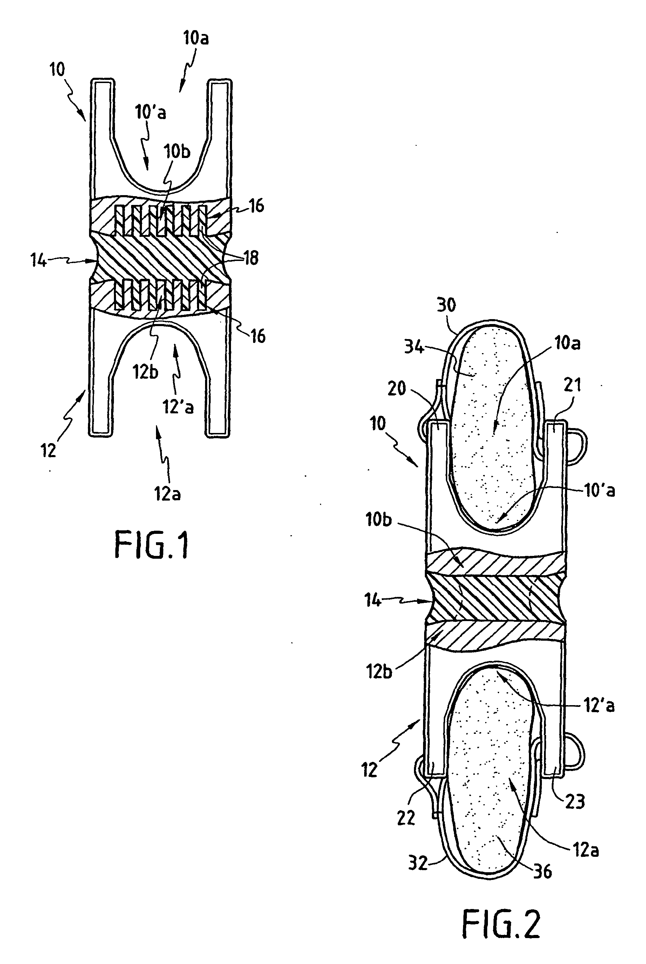

En se référant tout d'abord à la Figure 1 on décrira la cale et le mode de liaison des éléments qui la constituent.

L'implant intervertébral comporte deux éléments symétriques 10 et 12 présentant chacun une première extrémité 10a et 12a et une deuxième extrémité 10b et 12b. Les deux éléments 10 et 12 sont réalisés dans un matériau bio-compatible du type alliage de titane, de façon à être installés à demeure à l'intérieur du corps sur le rachis.

Chaque première extrémité 10a, 12a présente une gorge 10'a, 12'a dans laquelle une apophyse épineuse est apte à prendre appui, de façon que chaque première extrémité 10a, 12a entoure sensiblement la moitié du pourtour d'une apophyse, cette dernière traversant entièrement la première extrémité 10a, 12a.

Les éléments 10 et 12 sont reliés par une pièce de liaison 14, interposée entre eux, de façon que lesdits éléments 10 et 12 soient maintenus symétriquement l'un par rapport à l'autre. Ce sont précisément les deuxièmes extrémités 10b et 12b des éléments 10 et 12 qui sont reliées entre elles.

La pièce de liaison 14 est constituée d'un corps, obtenu par polymérisation, du type matière plastique. Ce corps est choisi parmi les matériaux dont la déformabilité élastique est supérieure à celle du matériau desdits éléments 10 et 12 et surtout dont les propriétés élastiques sont de l'ordre de celles des propriétés des ligaments postérieurs qui maintiennent les différents éléments du rachis.

Les composés organiques du silicium forment des polymères dont les propriétés mécaniques sont susceptibles d'être déterminées par le choix des composés de base, notamment par leur degré de substitution, la nature des substituants et leur poids moléculaire, et dont le comportement élastique est prépondérant par rapport au comportement plastique. Ainsi, ils constituent une famille de matériaux adaptés à la réalisation de la liaison entre lesdits deux éléments 10 et 12. En outre, ces polymères sont susceptibles de présenter une forte adhésivité sur les matériaux de composition minérale.- Ainsi, lorsque les éléments 10 et 12 sont réalisés en alliage de titane, la pièce de liaison 14 assure une bonne solidarisation.

Cependant, les matériaux de type polymères utilisables ne sont pas limités aux composés organiques du silicium et tout autre matériau présentant des propriétés comparables pourrait être utilisé.

Le matériau de ladite pièce de liaison 14 est apte à adhérer sur une paroi de solidarisation desdites deuxièmes extrémités 10b et 12b sensiblement plane. Cependant, afin d'accroître cette adhésivité, des évidements 16 sont ménagés dans la paroi de solidarisation des extrémités 10b et 12b et sont aptes à coopérer avec des aspérités 18 de la pièce de liaison 14 qui s'insèrent dans les évidements 16.

Cette caractéristique permet, d'une part d'augmenter la surface de contact entre les deux matériaux et ainsi d'augmenter la force de liaison entre eux selon une direction normale à ladite surface de contact, et d'autre part de créer des forces de frottement statique qui s'additionnent à la force d'adhérence.

Une telle liaison est réalisée soit en injectant le polymère à chaud entre les deux éléments 10 et 12 maintenus en regard dans un moule, soit en moulant le mélange de monomères à froid entre les deux éléments 10 et 12 si la vitesse de réaction est suffisamment lente. Ainsi, les aspérités 18 sont elles formées in situ, lorsque le polymère en phase liquide ou pâteuse, inséré dans les évidements 18, se solidifie après refroidissement ou après réaction chimique. On comprend que la pièce de liaison 14 est constituée par le polymère interposé entre les éléments 10 et 12 et que pour le maintenir entre ces éléments en regard lorsqu'il est à l'état liquide, les parois du moule doivent nécessairement entourer l'espace qui sépare les deux éléments 10 et 12 dans leur prolongement.

Selon un mode particulier de réalisation, non représenté, les évidements 16, ménagés dans la paroi de solidarisation, débouchent dans la paroi externe des éléments 10 et 12 afin que le polymère en phase liquide pénètre entièrement dans les évidements 16 sans que de l'air puisse y être emprisonné. De la sorte, la liaison entre le matériau de la pièce de liaison 14 et les éléments est renforcée.

Par ailleurs, les évidements, représentés parallèles à l'axe longitudinal de la cale sur la Figure 1, sont susceptibles d'être ménagés obliquement par rapport à cet axe longitudinal et/ou non rectiligne. Ces configurations permettent d'accroître les forces de frottement statique du polymère sur les éléments 10 et 12 ce qui renforce leur liaison.

Selon un autre mode de réalisation de l'invention, non représenté, les éléments 10 et 12 sont percés axialement dans la paroi de solidarisation de leur seconde extrémité 10b et 12b pour former un seul évidement débouchant dans leur première extrémité 10a et 12a au fond de leur gorge 10'a et 12'a. Une portion du perçage formant évidement situé à proximité de la gorge présente un diamètre supérieur au perçage qui débouche dans la paroi de solidarisation, de façon à former un épaulement. Ainsi, la pièce de liaison est moulée entre les éléments 10 et 12 de façon que le matériau du type polymère pénètre dans les deux évidements et les remplisse totalement jusqu'à la surface du fond de leur gorge 10'a et 12'a. En outre, le diamètre des perçages est supérieur au diamètre des évidements illustrés sur la Figure 1. Ainsi, après que le matériau du type polymère a retrouvé son état solide, non seulement il solidarise les deux éléments 10 et 12 par ses propriétés adhésives sur les parois internes des perçages, mais encore il les solidarise mécaniquement puisque les parties de matériau moulées dans lesdites portions de diamètres supérieurs viennent en butée contre lesdits épaulements.

On se référera maintenant aux Figures 2, 3 et 4 pour décrire des modes particuliers de mise en oeuvre de l'invention.

La Figure 2 illustre un premier mode particulier de mise en oeuvre de l'invention selon lequel les deux bords opposés des gorges 10'a et 12'a forment des ailes 20, 21, 22, 23 reliées deux à deux par des liens 30 et 32 dont la portion de longueur qui sépare lesdites ailes 20, 21 et 22, 23 est réglable et bloquée par la tension qui est susceptible de s'appliquer sur lesdits liens 30 et 32.

On retrouve sur la Figure 2 les deux éléments 10 et 12 reliés par la pièce de liaison 14. L'implant selon l'invention est inséré entre deux apophyses épineuses 34, 36 de deux vertèbres adjacentes de façon que les deux gorges 10'a et 12'a situées en opposition enserrent, partiellement, respectivement la portion sous-jacente de l'apophyse épineuse supérieure 34 et la portion sus-jacente de l'apophyse épineuse inférieure 36.

Les liens 30 et 32 recouvrent respectivement la portion sus-jacente de l'apophyse épineuse supérieure 34 et la portion sous-jacente de l'apophyse épineuse 36 de façon que les apophyses épineuses 34 et 36 soient aptes à être bloquées respectivement dans les gorges 10'a et 12'a. Le blocage s'effectue par le serrage des liens 30 et 32 qui, au moyen de fentes pratiquées dans les ailes 21 et 23, se coincent dans lesdites ailes 21 et 23.

Ainsi, les éléments 10 et 12 sont solidaires des deux apophyses 34 et 36 et le mouvement relatif desdites apophyses 34, 36 est possible dans les limites de déformation de ta pièce de liaison 14 en matériau de type polymère.

Lorsque les deux apophyses épineuses 34 et 36 se rapprochent l'une de l'autre, en particulier lors de l'extension du rachis; les deux éléments 10 et 12 compriment la pièce de liaison 14 de façon élastique, c'est-à-dire que la force qui tend à éloigner l'une de l'autre les apophyses épineuses 34 et 36 est sensiblement proportionnelle au déplacement relatif des deux apophyses. Ainsi, l'implant permet de suppléer le disque intervertébral ou une partie du disque intervertébral quant à l'espacement qu'il permet de maintenir entre deux vertèbres afin de ne pas coincer une quelconque racine. Il permet également d'obtenir des contraintes appliquées sur les apophyses épineuses qui sont compatibles avec les contraintes exercées par les ligaments postérieurs sur les apophyses.

En outre, lorsque les deux apophyses épineuses 34 et 36 s'éloignent l'une de l'autre, lors de la flexion du rachis, la pièce de liaison subit une traction dont la force de rappel est également sensiblement proportionnelle à l'allongement qu'elle subit, à tout le moins pour de faibles amplitudes. Ainsi, la flexion du rachis est réalisée avec une plus grande amplitude que celle qui est permise avec la solidarisation, de manière fixe, de deux apophyses épineuses.

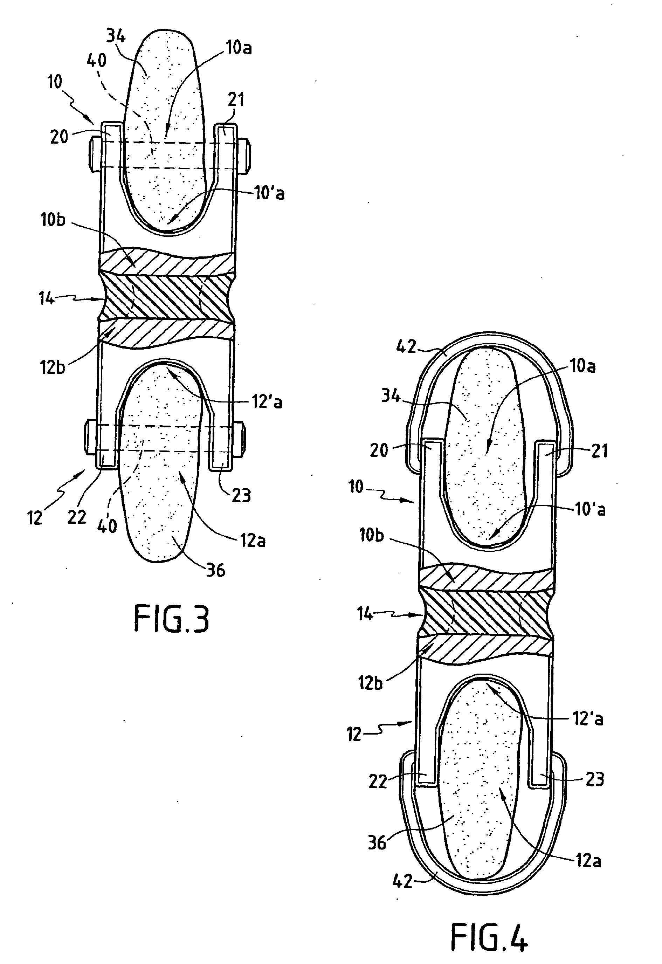

La Figure 3 illustre un deuxième mode particulier de mise en oeuvre de l'invention selon lequel les ailes des gorges et l'apophyse épineuse qui s'insère entre les deux, sont bloquées ensemble par une tige qui les traverse.

On retrouve sur la Figure 3 la cale intervertébrale entre les deux apophyses épineuses 34 et 36 et les paires d'ailes 20, 21 et 22, 23 qui enserrent partiellement les apophyses 34 et 36. En outre, les gorges 10'a et 12'a sont traversées par une tige 40 qui traverse également les apophyses épineuses 34 et 36. Les ailes 20, 21 et 22, 23 sont respectivement percées de deux orifices en regard à travers lesquels la tige 40 est apte à être insérée et fixée par ses extrémités.

En préalable à l'insertion de l'implant entre les apophyses épineuses 34, 36 ces dernières sont percées latéralement d'un orifice dont le diamètre est supérieur au diamètre de la tige 40. Ensuite, la cale intervertébrale est insérée entre les apophyses épineuses 34, 36, puis la tige 40 formant rivet est glissée de façon à traverser les ailes 21, 20 et 22, 23 ainsi que les apophyses épineuses 34, 36 correspondantes. Les extrémités des tiges 40 sont écrasées longitudinalement de manière à leur donner un diamètre supérieur à celui de l'orifice des ailes 21, 22, et 22, 23 qu'elles traversent. De cette façon, les tiges 40 sont bloquées en translation longitudinale vis-à-vis de la cale et, ainsi, les deux éléments 10 et 12 sont respectivement solidaires des apophyses épineuses 34 et 36.

Selon ce deuxième mode particulier de réalisation de l'invention, il n'est pas nécessaire de procéder à l'ablation des ligaments interépineux ni à la désinsertion du ligament supraépineux, sous-jacents et sus-jacents à l'espace intervertébral dans lequel la cale est insérée. En effet, lors de l'insertion de la cale, les paires d'ailes 20, 21 et 22, 23 n'étant pas reliées par des moyens aptes à entourer l'apophyse épineuse, seules sont nécessaires l'ablation des ligaments interépineux et la désinsertion du ligament supraépineux de l'espace intervertébral dans lequel la cale est insérée.

La Figure 4 illustre un troisième mode de mise en oeuvre de l'invention procurant les même avantages que ceux décrits ci-dessus pour le deuxième mode de mise en oeuvre.

On retrouve sur la Figure 4 la cale intervertébrale entre les deux apophyses épineuses 34 et 36 et les paires d'ailes 20, 21 et 22, 23 qui enserrent partiellement les apophyses 34 et 36. En revanche, les éléments 10 et 12 de la cale intervertébrale sont rendus solidaires, des apophyses épineuses 34 et 36 respectivement, au moyen d'une pièce hémicirculaire 42 formant agrafe.

Cette pièce hémicirculaire 42 comporte deux extrémités formant crochet dirigées vers l'intérieur de la pièce, aptes à être encastrées dans des orifices ménagés dans la paroi externe des paires d'ailes 20, 21 et 22, 23. La pièce 42 est élastiquement déformable de sorte qu'elle est fixable à force sur les éléments 10 et 12.

Après que la cale a été insérée entre les apophyses épineuses 34, 36, les agrafe 40 sont fixées sur les éléments 10 et 12 de façon que la paroi interne des pièces hémicirculaires 42, recouvre une portion de paroi des apophyses épineuses, complémentaire à la portion de paroi des apophyses épineuses recouverte par les gorges 10'a et 12'a. Ainsi, les apophyses épineuses 34 et 36 sont immobilisées dans les gorges 10'a et 12'a par les agrafes 42, rendant les éléments 10 et 12 solidaires desdites apophyses épineuses.

Les modes de réalisation et de mise en oeuvre de l'invention décrits ci-dessus ne sont pas uniquement limités à un implant intervertébral apte à être interposé entre deux vertèbres adjacentes. Ainsi on ne sortirait pas du cadre de l'invention en prévoyant un implant constitué de deux implants tels que décrits ci-dessus réunis par l'extrémité de leurs ailes suivant leur axe longitudinal de façon à limiter et à freiner le mouvement relatif de trois vertèbres consécutives.

## Revendications

1. Implant intervertébral apte à limiter et à freiner le mouvement relatif de deux vertèbres, comportant une cale destinée à s'appliquer entre les apophyses épineuses (34, 36) desdites vertèbres, ladite cale comprenant :
- deux éléments (10, 12) constitués dans un premier matériau, présentant une première extrémité (10a, 12a) solidarisable à une apophyse épineuse (34, 36) et une deuxième extrémité (10b, 12b) ; et,
- une pièce de liaison (14) constituée dans un deuxième matériau de plus grande déformabilité élastique que ledit premier matériau,
**caractérisé en ce que** ladite deuxième extrémité (10b, 12b) présente une paroi de solidarisation munie d'évidements (16), et **en ce que** ledit deuxième matériau est apte à adhérer audit premier matériau, ladite pièce de liaison (14) reliant les parois de solidarisation (10b, 12b) desdits deux éléments (10, 12) et présentant des aspérités (18) s'insérant dans lesdits évidements (16), de façon à augmenter les forces d'adhérence entre la pièce de liaison et lesdits deux éléments.

2. Implant intervertébral selon la revendication 1, **caractérisé en ce que** lesdits évidements (16) et lesdites aspérités (18) avec lesquelles ils coopèrent sont orientés sensiblement parallèlement à l'axe longitudinale de ladite cale.

3. Implant intervertébral selon la revendication 1 ou 2, **caractérisé en ce que** ledit deuxième matériau de ladite pièce de liaison (14) est constitué d'un corps obtenu par polymérisation.

4. Implant intervertébral selon l'une quelconque des revendications 1 à 3, **caractérisé en ce que** ledit premier matériau desdits éléments (10,12) est un alliage de titane.

5. Implant intervertébral selon l'une quelconque des revendications 1 à 4, **caractérisé en ce que** chaque première extrémité (10a, 12a) desdits deux éléments (10, 12) forme une gorge (10'a 12'a), définissant deux ailes (20, 21, 22, 23), apte à recevoir une apophyse épineuse (34, 36) et **en ce que** ledit implant comprend en outre un lien de longueur réglable (30, 32) reliant lesdites deux ailes (20, 21 ; 22, 23), ledit lien (30, 32) entourant une portion de ladite apophyse épineuse (34, 36) de façon à solidariser ladite première extrémité (10a 12a) à ladite apophyse épineuse (34, 36).

6. Implant intervertébral selon l'une quelconque des revendications 1 à 4, **caractérisé en ce que** chaque première extrémité (10a, 12a) desdits deux éléments forme une gorge (10'a, 12'a) apte à recevoir une apophyse épineuse (34, 36), ladite gorge définissant deux ailes (20, 21, 22, 23), et **en ce que** ledit implant comprend en outre une tige (40) apte à traverser latéralement lesdites ailes (20, 21, 22, 23) et ladite apophyse (34, 36) de façon à solidariser ladite première extrémité (10a, 12a) à ladite apophyse épineuse (34, 36).

7. Implant intervertébral selon l'une quelconque des revendications 1 à 4, **caractérisé en ce que** chaque première extrémité (10a, 12a) forme une gorge (10'a, 12'a) apte à recevoir une apophyse épineuse (34, 36), ladite gorge (10'a , 12'a) définissant deux ailes (20, 21, 22, 23), et **en ce que** ledit implant comprend en outre une pièce hémicirculaire (42) formant agrafe reliant lesdites ailes (20, 21, 22, 23), ladite agrafe (42) entourant une portion de ladite apophyse épineuse (34, 36) de façon à solidariser ladite première extrémité (10a 12a) à ladite apophyse épineuse (34, 36).

## Claims

1. An intervertebral implant suitable for limiting and slowing down the relative movements of two vertebrae, comprising a spacer designed to be applied between two spinous processes (34, 36) of said vertebrae, said spacer comprising:
- two elements (10, 12) made of a first material, and each presenting a first end (10a, 12a) and a second end (10b, 12b), said first end (10a, 12a) being securable to a spinous process (34, 36); and
- a connection piece (14) made of a second material of greater elastic deformability than said first material,
**characterised in that** said second end (10b, 12b) presents a securing wall provided with recesses (16), and **in that** said second material is suitable for bonding onto said first material, said connection piece (14) interconnecting the securing walls (10b, 12b) of said two elements (10, 12) and presenting projections (18) fitting in said recesses (16) in such a manner as to increase bonding forces between the connection piece and said two elements.

2. An intervertebral implant according to claim 1, **characterised in that** said recesses (16) and said projections (18) with which the former cooperate are substantially oriented in parallel to the longitudinal axis of said spacer.

3. An intervertebral implant according to claim 1 or 2, **characterised in that** said second material of said connection piece (14) is constituted by a body obtained by polymerization.

4. An intervertebral implant according to any one of claims 1 to 3, **characterised in that** said first material of said elements (10, 12) is a titanium alloy.

5. An intervertebral implant according to any one of claims 1 to 4, **characterised in that** each first end (10a, 12a) of said two elements (10, 12) forms a notch (10'a, 12'a) defining two wings (20, 21, 22, 23), which is suitable for receiving a spinous process (34, 36), and **in that** said implant further comprises a tie of adjustable length (30, 32) interconnecting said two wings (20, 21, 22, 23), said tie (30, 32) surrounding a portion of said spinous process (34, 36) in such a manner as to secure said first end (10a, 12a) to said spinous process (34, 36).

6. An intervertebral implant according to any one of claims 1 to 4, **characterised in that** each first end (10a, 12a) of said two elements forms a notch (10'a, 12'a), which is suitable for receiving a spinous process (34, 36), said notch defining two wings (20, 21, 22, 23), and **in that** said implant further comprises a pin (40) that is suitable for passing laterally through said wings (20, 21, 22, 23) and said process (34, 36) in such a manner as to secure said first end (10a, 12a) to said spinous process (34, 36).

7. An intervertebral implant according to any one of claims 1 to 4, **characterised in that** each first end (10a, 12a), which is suitable for receiving a spinous process (34, 36), said notch defining two wings (20, 21, 22, 23), and **in that** said implant further comprises a clip-forming semicircular part (42) interconnecting said wings (20, 21, 22, 23), said clip (42) surrounding a portion of said spinous process (34, 36) in such a manner as to secure said first end (10a, 12a) to said spinous process (34, 36).

## Patentansprüche

1. Zwischenwirbelimplantat, das die relative Bewegung zweier Wirbel bremsen kann, umfassend einen Keil, der dazu bestimmt ist, zwischen den Knochenfortsätzen (34, 36) der Wirbel angelegt zu werden, wobei der Keil umfaßt:
- zwei Elemente (10, 12), die aus einem ersten Material bestehen und ein erstes Ende (10a, 12a), das mit einem Knochenfortsatz (34, 36) verbunden werden kann, und ein zweites Ende (10b, 12b) aufweisen; und
- ein Verbindungsstück (14), das aus einem zweiten Material mit einer größeren elastischen Verformbarkeit als das erste Material besteht,
**dadurch gekennzeichnet, daß** das zweite Ende (10b, 12b) eine Verbindungswand, die mit Aussparungen (16) versehen ist, aufweist, und daß das zweite Material an dem ersten Material anhaften kann, wobei das Verbindungsstück (14) die Verbindungswände (10b, 12b) der beiden Elemente (10, 12) verbindet und Rauhigkeiten (18) aufweist, die sich in die Aussparungen (16) einfügen, um die Haftkräfte zwischen dem Verbindungsstück und den beiden Elementen zu erhöhen.

2. Zwischenwirbelimplantat nach Anspruch 1, **dadurch gekennzeichnet, daß** die Aussparungen (16) und die Rauhigkeiten (18), mit denen sie zusammenwirken, im Wesentlichen parallel zur Längsachse des Keils ausgerichtet sind.

3. Zwischenwirbelimplantat nach Anspruch 1 oder 2, **dadurch gekennzeichnet, daß** das zweite Material des Verbindungsstücks (14) aus einem durch Polymerisation hergestellten Körper gebildet ist.

4. Zwischenwirbelimplantat nach einem der Ansprüche 1 bis 3, **dadurch gekennzeichnet, daß** das erste Material der Elemente (10, 12) eine Titanlegierung ist.

5. Zwischenwirbelimplantat nach einem der Ansprüche 1 bis 4, **dadurch gekennzeichnet, daß** jedes erste Ende (10a, 12a) der beiden Elemente (10, 12) eine Rille (10'a, 12'a) bildet, die zwei Flügel (20, 21, 22, 23) definiert, die einen Knochenfortsatz (34, 36) aufnehmen können, und daß das Implantat ferner ein Bindemittel von einstellbarer Länge (30, 32) umfaßt, das die beiden Flügel (20, 21; 22, 23) verbindet, wobei das Bindemittel (30, 32) einen Abschnitt des Knochenfortsatzes (34, 36) umgibt, um das erste Ende (10a, 12a) mit dem Knochenfortsatz (34, 36) zu verbinden.

6. Zwischenwirbelimplantat nach einem der Ansprüche 1 bis 4, **dadurch gekennzeichnet, daß** jedes erste Ende (10a, 12a) der beiden Elemente eine Rille (10'a, 12'a) bildet, die einen Knochenfortsatz (34, 36) aufnehmen kann, wobei die Rille zwei Flügel (20, 21, 22, 23) definiert, und daß das Implantat ferner eine Stange (40) umfaßt, die seitlich die Flügel (20, 21, 22, 23) und die Apophyse (34, 36) queren kann, um das erste Ende (10a, 12a) mit dem Knochenfortsatz (34, 36) zu verbinden.

7. Zwischenwirbelimplantat nach einem der Ansprüche 1 bis 4, **dadurch gekennzeichnet, daß** jedes erste Ende (10a, 12a) eine Rille (10'a, 12'a) bildet, die einen Knochenfortsatz (34, 36) aufnehmen kann, wobei die Rille (10'a, 12'a) zwei Flügel (20, 21, 22, 23) definiert, und daß das Implantat ferner ein halbkreisförmiges Stück (42) umfasst" das eine die beiden Flügel (20, 21, 22, 23) verbindende Klammer bildet, wobei die Klammer (42) einen Abschnitt des Knochenfortsatzes (34, 36) umgibt, um das erste Ende (10a, 12a) mit dem Knochenfortsatz (34, 36) zu verbinden.
